(19) Europäisches Patentamt
European Patent Office
Office européen des brevets

(11) Numéro de publication : 0 289 371 B1

(12) # FASCICULE DE BREVET EUROPEEN

(45) Date de publication du fascicule du brevet : 25.09.91 Bulletin 91/39

(51) Int. Cl.[5] : C07D 471/04, A61K 31/435, // (C07D471/04, 235:00, 221:00)

(21) Numéro de dépôt : 88400666.9

(22) Date de dépôt : 21.03.88

(54) **Dérivés d'imidazopyridines, leur préparation et leur application en thérapeutique.**

Le dossier contient des informations techniques présentées postérieurement au dépôt de la demande et ne figurant pas dans le présent fascicule.

(30) Priorité : 27.03.87 FR 8704276
27.03.87 FR 8704277

(43) Date de publication de la demande : 02.11.88 Bulletin 88/44

(45) Mention de la délivrance du brevet : 25.09.91 Bulletin 91/39

(84) Etats contractants désignés : AT BE CH DE ES FR GB GR IT LI LU NL SE

(56) Documents cités :
EP-A- 0 050 563
FR-A- 2 599 368
CHEMICAL SOCIETY REVIEWS 18 (1979), 563-565

(73) Titulaire : SYNTHELABO
58 rue de la Glacière
F-75013 Paris (FR)

(72) Inventeur : George, Pascal
39, Henri de Vilmorin
F-94400 Vitry sur Seine (FR)
Inventeur : Allen, John
3, Square Lebrun
F-78180 Voisins le Bretonneux (FR)
Inventeur : Jaurand, Guy
41, rue Jean Raynal
F-91390 Morsang sur Orge (FR)
Inventeur : De Peretti, Danièle
14, rue Ampère
F-92160 Antony (FR)

(74) Mandataire : Thouret-Lemaitre, Elisabeth et al
SYNTHELABO Service Brevets 22, avenue Galilée
F-92352 LE PLESSIS ROBINSON CEDEX (FR)



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

**Description**

La présente invention a pour objet des dérivés d'imidazopyridines, leur préparation et leur application en thérapeutique.

Des imidazo[1,2-a]pyridines ont déjà été décrites dans la littérature, par exemple dans les demandes de brevets EP-A-50563 et FR-A-2599368.

Les composés de l'invention répondent à la formule (I)

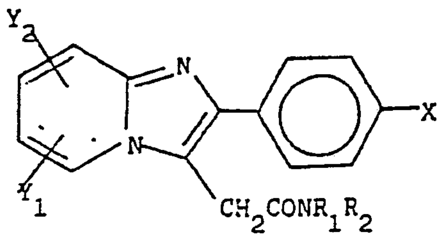

(I)

dans laquelle

- soit $Y_1$ représente un atome d'halogène,

  $Y_2$ représente soit un radical SR dans lequel R est H ou un radical $(C_{1-4})$ alkyle ou un radical (alcoxy-4 phényl)méthyle, soit un radical hydroxy, soit un radical $(C_{1-4})$alcoxy ; X est un atome d'halogène,

  et $R_1$ et $R_2$ représentent chacun, indépendamment l'un de l'autre, soit un atome d'hydrogène, soit un radical $(C_{1-4})$alkyle droit ou ramifié,

- soit $Y_1$ représente un atome d'hydrogène,

  $Y_2$ représente un radical SR dans lequel R est H ou un radical $(C_{1-4})$alkyle ou un radical (alcoxy-4 phényl-)méthyle, soit un radical hydroxy, soit un radical $(C_{1-4})$alcoxy ; X est un atome d'halogène ou un radical $(C_{1-4})$alkyle,

  et $R_1$ et $R_2$ représentent chacun, indépendamment l'un de l'autre, soit un atome d'hydrogène, soit un radical $(C_{1-4})$alkyle droit ou ramifié.

Les composés préférés de l'invention sont ceux pour lesquels
$Y_1$ est en position 6 et représente un atome d'halogène,
$Y_2$ est en position 8 et représente soit un radical SR dans lequel R est H, un radical $(C_{1-4})$alkyle ou un radical (alcoxy-4 phényl)méthyle, soit un radical hydroxy, soit un radical $(C_{1-4})$alcoxy ;
X est un atome d'halogène ou un radical $(C_{1-4})$alkyle,
$R_1$ et $R_2$ représentent chacun, indépendamment l'un de l'autre, soit un atome d'hydrogène, soit un radical $(C_{1-4})$alkyle droit ou ramifié,
plus particulièrement ceux pour lesquels $Y_1$ est en position 6 et représente un atome de chlore,
$Y_2$ est en position 8 et représente un radical méthylthio, mercapto, méthoxy, hydroxy, n-butylthio ou (méthoxy-4 phényl)méthylthio,
X est un atome de chlore ou le radical méthyle,
$R_1$ et $R_2$ représentent chacun le radical méthyle ou le radical n-propyle.
D'autres composés présentant un intérêt sont ceux pour lesquels $Y_1$ est H,
$Y_2$ est en position 6 et représente un radical Salkyle ou alcoxy,
X est un atome d'halogène ou un radical $(C_{1-4})$alkyle,
$R_1$ et $R_2$ représentent chacun, indépendamment l'un de l'autre, soit un atome d'hydrogène, soit un radical $(C_{1-4})$alkyle droit ou ramifié,
plus particulièrement ceux pour lesquels $Y_1$ est H,
$Y_2$ est en position 6 et représente le radical méthylthio ou méthoxy,
X est un atome de chlore ou le radical méthyle,
$R_1$ et $R_2$ représentent chacun le radical méthyle ou le radical n-propyle.

Les composés de l'invention sont préparés par des procédés de préparation différents selon les substituants $Y_1$ et/ou $Y_2$.

Les composés de l'invention dans lesquels $Y_2$ est 8-SR sont préparés selon le schéma réactionnel donné en annexe 1 :
on brome une amino-2 pyridine de formule (II) puis on fait réagir le composé bromé (III) avec une bromocétone de formule (IV) ; on fait ensuite réagir l'imidazopyridine obtenue de formule (V) avec un acétal de formule (VI), on traite le composé obtenu de formule (VII) par $SOCl_2$ puis on réduit le composé chloré obtenu in situ avec un agent réducteur tel que la Rongalite[R] ; on fait réagir le composé (VIII) avec un alkylthiolate de sodium préparé

dans du diméthylformamide, à partir d'un alkylthiol et d'hydrure de sodium, on obtient alors un dérivé S-alkylé (I).

On prépare le composé (I) dans lequel $Y_2$ est 8-SH à partir du composé (VIII) : on fait réagir ce composé (VIII) avec le méthoxy-4 benzèneméthanethiol dans du diméthylformamide en présence d'hydrure de sodium pour obtenir un composé portant un radical (méthoxy-4 benzyl)-thio à la place de l'atome de brome. On agite ce composé à 0°C dans de l'acide trifluoroacétique et en présence d'acétate de mercure pour obtenir le thiol (I), sous forme de thiolate de mercure.

On peut également isoler le composé sous forme de disulfure à partir du thiolate de mercure par action de l'acide trifluoroacétique et du sulfure d'hydrogène.

Les composés de l'invention dans lesquels $Y_2$ est 8-alcoxy et 8-OH sont préparés selon le schéma réactionnel de l'annexe 2 :
on fait réagir un composé (V) dont la préparation est décrite dans l'annexe 1 avec un alcoxylate de sodium dans de l'hexaméthylphosphorotriamide, puis on fait réagir le composé (X) avec du diéthoxy-1,1 N,N-$R_1R_2$ acétamide, on traite le composé obtenu (XI) par $SOCl_2$ puis par de la Rongalite$^R$ pour le transformer en composé alcoxylé (I) ; on obtient le composé (I) correspondant dans lequel $Y_2$ est OH par action de $BBr_3$ sur le composé alcoxylé (I).

Les composés de l'invention dans lesquels $Y_2$ est 6-alcoxy ou 6-SR sont préparés selon le schéma réactionnel donné dans l'annexe 3 : on condense une amino-2 pyridine de formule (XII) avec une α-bromo acétophénone de formule (XIII) puis on condense l'imidazopyridine de formule (XIV) obtenue avec un acétal de glyoxamide de formule (VI) ; on traite l'α-hydroxyamide (XV) par $SOCl_2$ puis on réduit le composé obtenu à l'aide de Rongalite pour obtenir le composé de formule (I). Les alcoxy-5 pyridinamines (XII) sont obtenues selon la méthode décrite par G.J. Clark, L.W. Deady, Aust. J. Chem. 34, 927 (1981).
Les alkylthio-5 pyridinamines (XII) sont obtenues selon la méthode décrite par Zelinskii Org. Chem. Inst. Derwent 79-88949 B (49).

Les exemples suivants illustrent l'invention.
Les analyses et les spectres IR et RMN confirment la structure des composés.

Exemple 1. Chloro-6 (chloro-4 phényl)-2 méthylthio-8 N,N-dipropyl-imidazo[1,2-a]pyridine-3-acétamide.

1.1. Amino-2 bromo-3 chloro-5 pyridine.

On fait réagir 20 g (77,8 mmoles) d'amino-2 chloro-5 pyridine dissous dans 160 ml de $CH_2Cl_2$ avec 8 ml de brome que l'on ajoute goutte à goutte, à 0°C. Après la fin de l'addition, on laisse le mélange réagir à 25°C pendant 2 h. On lave la suspension avec une solution de soude à 10%, on lave la phase organique à l'eau puis la sèche sur $MgSO_4$, on filtre et évapore. On fait recristalliser le composé obtenu dans de l'isopropanol. F = 82°C.

1.2. Bromo-8 chloro-6 (chloro-4 phényl)-2 imidazo[1,2-a]pyridine.

On fait réagir 17 g (82 mmoles) d'amino-2 bromo-3 chloro-5 pyridine, en solution dans 150 ml d'éthanol, avec 29 g de bromo-2 (chloro-4 phényl)-1 éthanone-1 et 14 g d'hydrogénocarbonate de sodium. On porte le mélange à la température du reflux pendant 6 h et on le refroidit. On filtre le précipité, le rince avec $CH_2Cl_2$, concentre le filtrat sous pression réduite. Après recristallisation du résidu d'évaporation dans de l'acétate d'éthyle, le composé fond à 178°C.

1.3. Bromo-8 chloro-6 (chloro-4 phényl)-2 α-hydroxy N,N-dipropyl-imidazo[1,2-a]pyridine-3-acétamide.

On prépare une solution de 2,03 g de diéthoxy-1,1 N,N-dipropyl-acétamide dans 30 ml d'acide acétique glacial et 0,1 ml d'acide chlorhydrique à 37%. On porte la solution à 60°C pendant 2 h. On ajoute à la solution 312 mg d'acétate de sodium puis après 10 mn, 2,9 mmoles du composé obtenu précédemment sous 1.2. On porte le mélange réactionnel à 60°C pendant 3 h environ, on évapore l'acide acétique, on ajoute de l'eau puis on extrait le mélange avec du $CH_2Cl_2$. On sèche la phase organique sur $MgSO_4$, filtre et concentre le filtrat sous pression réduite. Après recristallisation du résidu d'évaporation dans l'éthanol le composé fond à 199-200°C.

1.4. Bromo-8 chloro-6 (chloro-4 phényl)-2 N,N-dipropyl-imidazo[1,2-a]pyridine-3-acétamide.

On fait réagir 5 g (10 mmoles) du composé obtenu sous 1.3 en solution dans 100 ml de $CH_2Cl_2$ avec 2,2

ml de $SOCl_2$. On chauffe le mélange réactionnel à 60°C pendant 2 h. On évapore à sec sous pression réduite, on reprend le résidu avec du $CHCl_3$, et évapore à nouveau à sec puis on sèche le résidu sous vide.

On dissout le composé obtenu dans du $CH_2Cl_2$ et ajoute 4,7 g de Rongalite ; on maintient le mélange à 20°C pendant 48 h. On ajoute de l'eau pour dissoudre le solide formé, décante la phase organique et la lave à l'eau, puis à la soude à 30%, puis à l'eau. On sèche la phase organique sur $MgSO_4$, filtre et concentre le filtrat sous pression réduite. On fait recristalliser le résidu d'évaporation dans de l'éthanol. F = 189-192°C.

1.5. Chloro-6 (chloro-4 phényl)-2 méthylthio-8 N,N-dipropyl-imidazo[1,2-a]pyridine-3-acétamide.

On prépare une solution 5,8 molaire de méthanethiol dans du tétrahydrofuranne. On mélange, à 20°C, 1,8 ml de cette solution, 2 ml de diméthylformamide (DMF), et 480 mg d'hydrure de sodium à 50% dans l'huile. On ajoute lentement une solution de 2,5 g (5,18 mmoles) du composé obtenu sous 1.4 dans 25 ml de DMF. On agite le mélange à 20°C pendant 1 h 30, puis à 60°C pendant 1 h. On ajoute de l'eau, décante la phase organique, la sèche sur $MgSO_4$, filtre et évapore le filtrat sous pression réduite. On purifie le résidu d'évaporation par chromatographie sur colonne de silice puis fait recristalliser le produit dans de l'isopropanol. F = 152-154°C.

Exemple 2. Chloro-6 (chloro-4 phényl)-2 [[(méthoxy-4 phényl)méthyl]thio]-8 et mercapto-8 N,N-dipropyl-imidazo[1,2-a]pyridine-3-acétamides.

2.1. Dans un ballon, on introduit, tout en agitant, sous argon, de l'hydrure de sodium et du diméthylformamide puis, goutte à goutte, 2 équivalents de méthoxy-4 benzèneméthanethiol. On laisse le mélange réagir pendant 50 mn. On introduit 345 mg (0,17 mmole) du composé obtenu sous 1.4. Après 1 h on porte le mélange réactionnel à 40°C pendant 1 heure.

On le laisse refroidir, on ajoute de la glace et de l'eau, on extrait 3 fois à l'éther, lave la phase organique à l'eau, la sèche sur $Na_2SO_4$, filtre et concentre le filtrat sous pression réduite. On fait recristalliser le produit obtenu dans un mélange dichlorométhane/éther. F = 125-126°C.

2.2. On introduit 111 mg (0,2 mmole) du composé obtenu sous 2.1. dans 1 ml d'acide trifluoroacétique en présence d'anisole (20 µl). On traite avec 63,8 mg (0,2 mmole) d'acétate de mercure, tout en agitant, à 0°C, pendant 15 mn. On isole le composé formé et le purifie par chromatographie sur silice avec l'éluant chloroforme/méthanol 95/5. Le composé obtenu sous forme de thiolate de mercure fond à 191-192°C.

On peut également isoler le composé sous forme de disulfure à partir du thiolate de mercure par action de l'acide trifluoroacétique et du sulfure d'hydrogène. F = 126-128°C.

Exemple 3. Chloro-6 (chloro-4 phényl)-2 méthoxy-8 N,N-dipropyl-imidazo[1,2-a]pyridine-3-acétamide.

3.1. Chloro-6 (chloro-4 phényl)-2 méthoxy-8 imidazo[1,2-a]pyridine.

On ajoute 10 g (29,4 mmoles) de bromo-8 chloro-6 (chloro-4 phényl)-2 imidazo[1,2-a]pyridine, préparée sous 1.2., en solution dans 60 ml d'hexaméthylphosphorotriamide, à une solution de méthylate de sodium préparé au moyen de 2,03 g de sodium et de 15 ml de méthanol, on porte le mélange réactionnel à 60°C pendant 2 h 30, on ajoute de l'eau, extrait avec du $CH_2Cl_2$, lave la phase organique à l'eau, sèche sur $MgSO_4$, filtre et concentre le filtrat sous pression réduite. On fait recristalliser le résidu d'évaporation dans un mélange acétate d'éthyle/hexane. F = 150-151°C.

3.2. Chloro-6 (chloro-4 phényl)-2 α-hydroxy méthoxy-8 N,N-dipropyl-imidazo[1,2-a]pyridine-3-acétamide.

On prépare une solution de 9,46 g de diéthoxy-1,1 N,N-dipropyl acétamide dans 135 ml d'acide acétique glacial et 0,46 ml d'acide chlorhydrique à 37%.

On chauffe le mélange réactionnel à 60°C pendant 2 h.

On ajoute 1,3 g d'acétate de sodium puis, après 10 mn, 4 g (13,6 mmoles) du composé obtenu sous 3.1.

On chauffe le mélange réactionnel à 60°C pendant 3 h puis on le refroidit et on évapore à sec.

On reprend le résidu avec du $CH_2Cl_2$, lave la phase organique à l'eau, sèche sur $MgSO_4$, filtre et concentre le filtrat sous pression réduite. On purifie le produit obtenu par chromatographie avec l'éluant hexane/acétate d'éthyle 3/1. Le composé obtenu est utilisé tel quel pour l'étape suivante.

3.3. Chloro-6 (chloro-4 phényl)-2 méthoxy-8 N,N-dipropyl-imidazo[1,2-a]pyridine-3-acétamide.

On mélange 2,75 g (6,12 mmoles) du composé obtenu précédemment, 50 ml de $CH_2Cl_2$ et 1,34 ml de $SOCl_2$. On porte le mélange réactionnel à 60°C pendant 2 h, on évapore à sec puis sèche sous vide. On ajoute 30 ml de $CH_2Cl_2$ pur puis 2,82 g de Rongalite. On laisse le mélange réactionnel à 20°C pendant une nuit.

On ajoute de l'eau au mélange, décante la phase organique, la lave à l'eau puis avec une solution de chlorure de sodium, sèche sur $MgSO_4$, filtre et concentre le filtrat sous pression réduite.

On purifie le résidu par chromatographie (éluant : hexane 2/acétate d'éthyle 1).

F = 162-163°C.

Exemple 4. Chloro-6 (chloro-4 phényl)-2 hydroxy-8 N,N-dipropyl-imidazo[1,2-a]pyridine-3-acétamide.

A 0,15 mg (0,36 mmole) du produit obtenu sous 3.3. en solution dans 15 ml de dichlorométhane, on ajoute 0,2 ml (0,53 g, 2,11 mmoles) de $BBr_3$ à –60°C. On laisse le mélange revenir à la température ambiante, sous agitation, durant deux heures.

L'excès de $BBr_3$ est alors détruit par du méthanol à –78°C. On évapore le mélange réactionnel, purifie le résidu obtenu par chromatographie (éluant : dichlorométhane/méthanol 95/5). On obtient un produit que l'on fait cristalliser sous forme de base dans de l'acétate d'éthyle.

F = 190-191°C.

Exemple 5. Butylthio-8 chloro-6 (chloro-4 phényl)-2 N,N-dipropyl-imidazo[1,2-a]pyridine-3-acétamide.

Dans un ballon tricol, sous agitation magnétique, sous argon, et refroidi à l'aide d'un bain de glace, on introduit 300 mg (2éq) d'hydrure de sodium à 50% dans de l'huile, 30 ml de diméthylformamide, puis, goutte à goutte, 600 µl (2éq) de butanethiol et on agite pendant 45 mn. A l'aide d'une ampoule à brome, on ajoute 1,5 g (3,10 mmole) de bromo-8 chloro-6 (chloro-4 phényl)-2 N,N-dipropyl imidazo[1,2-a]pyridine-3-acétamide dans 30 ml de diméthylformamide. On laisse revenir le mélange à la température ambiante et on agite pendant 3 heures.

Après refroidissement, on ajoute lentement de la glace puis de l'eau.

On extrait 3 fois à l'éther, on lave une fois à l'eau, on sèche sur sulfate de magnésium, on filtre et on évapore à sec.

Après chromatographie sur gel de silice, avec l'éluant $CH_2Cl_2$ 99,5/méthanol 0,5, le composé fond à 119-120°C.

Exemple 6. (Chloro-4 phényl)-2 méthoxy-6 N,N-diméthyl-imidazo[1,2-a] pyridine-3-acétamide.

6.1. (Chloro-4 phényl)-2 méthoxy-6 imidazo[1,2-a]pyridine.

Sous argon on chauffe 4 h 30, au reflux, un mélange de 1,3 g (10,5 mmole) de méthoxy-5 pyridamine-2, 2,44 g (1éq.) de α-bromo chloro-4 acétophénone et 1,76 g (2eq) de bicarbonate de sodium dans 20 ml d'alcool à 95%. On évapore à sec, reprend entre $CH_2Cl_2$ et $H_2O$, lave, sèche et évapore.

Le produit est purifié par chromatographie puis cristallisé dans de l'éther.

F = 148-9°C.

La méthoxy-5 pyridinamine-2 a été obtenue selon un procédé décrit dans la littérature : G.J. Clark, L. W. Deady, Aust. J. Chem. 34, 927 (1981).

6.2. (Chloro-4 phényl)-2 α-hydroxy méthoxy-6 N,N-diméthyl-imidazo[1,2-a]pyridine-3-acétamide.

On prépare une solution de 4,95 g (26,7 mmole) diéthoxy-1,1 N,N-diméthylacétamide dans 90 ml d'acide acétique. On chauffe à 50°C, ajoute 0,7 ml d'acide chlorhydrique à 37% et agite 2 h à cette température. On ajoute 2,2 g (27,7 mmole) d'acétate de sodium puis, après 15 mn, 2,3 g (8,9 mmole) du composé obtenu en 6.1. On continue de chauffer pendant 2 h. On évapore à sec, reprend entre $CH_2Cl_2$ et de l'eau ammoniaquée, lave la phase organique, sèche, évapore et cristallise dans de l'oxyde d'éthyle. On obtient 2,2 g de solide blanc.

F = 185-186°C (dec).

Le composé contient 0,7% d'eau.

6.3. (Chloro-4 phényl)-2 méthoxy-6 N,N-diméthyl-imidazo[1,2-a] pyridine-3-acétamide.

On dissout 2,1 g (5,8 mmole) du composé obtenu en 6.2 dans 115 ml de $CH_2Cl_2$, ajoute 11,5 ml de chlorure

de thionyle et agite 15 h à la température ambiante. Puis on évapore à sec, reprend le solide résiduel au pentane, et sèche sous vide le solide obtenu. On dissout 2,4 g de ce composé dans 180 ml de $CH_2Cl_2$, on ajoute 2,67 g (3 eq) de Rongalite et agite 20 h à la température ambiante. On filtre le solide obtenu et le purifie par chromatographie puis on le fait cristalliser dans de l'éther.

F = 170-2°C (dec).

Les composés pour lesquels $Y_2$ est $SCH_3$ en 6 sont préparés selon le même mode opératoire : à partir de la méthylthio-5 pyridinamine, selon le schéma de l'annexe 3.

Les composés préparés à titre d'exemples sont représentés dans le tableau suivant :

Tableau

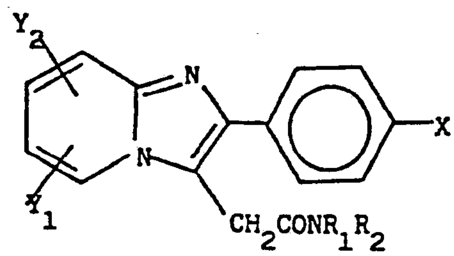

| Composé | $Y_1$ | $Y_2$ | X | $R_1$=$R_2$ | F(°C) |
|---|---|---|---|---|---|
| 1 | 6-Cl | 8-$SCH_3$ | Cl | $nC_3H_7$ | 152-154 |
| 2 | 6-Cl | 8-SH | Cl | $nC_3H_7$ | 191-192* |
| 3 | 6-Cl | 8-$OCH_3$ | Cl | $nC_3H_7$ | 162-163 |
| 4 | 6-Cl | 8-OH | Cl | $nC_3H_7$ | 190-191 |
| 5 | 6-Cl | 8-$SC_4H_9$n | Cl | $nC_3H_7$ | 119-120 |
| 6 | 6-Cl | 8-$SCH_2$-$C_6H_4OCH_3$ | Cl | $nC_3H_7$ | 125-126 |
| 7 | H | 6-$OCH_3$ | Cl | $CH_3$ | 170-172 |
| 8 | H | 6-$SCH_3$ | $CH_3$ | $CH_3$ | 119-121 |
| 9 | H | 6-$SCH_3$ | Cl | $CH_3$ | 146-147 |

* thiolate de $Hg^{++}$

Les composés de l'invention ont été soumis à des essais pharmacologiques qui ont montré leurs intéressantes propriétés pharmacologiques dans divers domaines.

La toxicité des composés a été déterminée chez les souris par voie intrapéritonéale.

La DL 50 va de 500 à 1000 mg/kg.

L'activité sédative ou hypnotique a été déterminée en observant l'action des composés sur l'ECoG du rat curarisé (Depoortere H., Rev. E.E.G. Neurophysiol., (1980) 10, 3, 207-214). Chez le rat immobilisé, les produits à étudier sont injectés par voie intrapéritonéale ou orale aux doses croissantes de 1 à 30 mg/kg. Ils induisent des tracés de sommeil aux doses égales ou supérieures à 0,3 mg/kg i.p.

L'activité anticonvulsivante des composés a été déterminée selon le test de l'inhibition des convulsions cloniques induites par le pentétrazol chez la souris selon la méthode de Worms et coll (J. Pharmacol. Exp. Ther., 220 : 660-671). Chez des souris mâles (20-22 g) CD1 Charles River, les convulsions cloniques sont induites par injection i.v. de 35 mg/kg de pentetrazol, 30 mn après l'injection i.p. du produit à tester.

La DA 50 est la dose qui protège 50% des animaux des convulsions cloniques provoquées par le pentétrazol. La DA 50 des composés de l'invention va de 0,1 à 10 mg/kg.

Effets sur la durée du "sommeil" induit par le hydroxy-4 butyrate de sodium.

Cette action a été déterminée par l'influence d'un composé sur la durée du "sommeil" induit par le hydroxy-4 butyrate de sodium (GBH) chez le rat curarisé.

Les animaux utilisés sont des rats mâles de souche Charles River de 200 ± 20 g. Les animaux, immobilisés par l'alloférine à raison de 5 mg/kg par voie i.p., sont placés sous respiration artificielle à l'aide d'un masque appliqué sur le museau (fréquence respiratoire = 50/minute ; volume respiratoire = 14 ml).

L'oesophage est préalablement ligaturé afin d'éviter l'entrée de l'air dans l'estomac.

Des électrodes corticales front-pariétales et occipitales permettent l'enregistrement de l'acitivité électrocorticographique sur un polygraphe Grass modèle 79 P à la vitesse de 6 mm/sec.

La préparation de l'animal est effectuée sous anesthésie locale (xylocaïne à 2%). Les rats sont maintenus tout au long de l'expérience à température constante (37,5°C). Dix minutes après la fin de la préparation du rat, une dose de 200 mg/kg de hydroxy-4 butyrate de sodium est injectée par voie intraveineuse au niveau de la queue.

Une dose de 10 mg/kg du composé à étudier est administrée par voie intrapéritonéale 3 minutes après l'administration du hydroxy-4 butyrate de sodium.

L'évaluation des tracés s'effectue par périodes de 15 minutes durant 75 minutes après l'injection de GHB. Durant cette période d'analyse, la durée totale du "sommeil" est déterminée. Une série de 15 témoins permet de préciser la durée du "sommeil GHB".

L'analyse statistique des résultats est réalisée à l'aide du test "U" de Mann-Whitney.

Certains composés réduisent les effets du GHB (jusqu'à 40% de diminution de la durée du sommeil à la dose de 10 mg/kg), tandis que d'autres potentialisent ces effets (jusqu'à 40% d'augmentation à la dose de 10 mg/kg). On constate également que les effets peuvent être opposés selon que les composés sont administrés à fortes doses ou à faibles doses.

Les résultats des essais pharmacologiques montrent que les composés de l'invention sont actifs dans le domaine du système nerveux central et possèdent des propriétés anxiolytiques, inductrices de sommeil, hypnotiques et anticonvulsivantes ; les composés de l'invention sont utiles pour le traitement des états d'anxiétés, des troubles du sommeil et autres affections neurologiques et psychiatriques.

Les composés de l'invention présentent également une très haute affinité pour les sites de liaison de type périphérique des benzodiazépines ($\omega_3$). Cette activité est déterminée selon la méthode décrite dans la littérature par S. Arbilla, H. Depoortere, P. George, S.Z. Langer, Naunyn Schmiedeberg's Archiv. Pharmacol 330, 248-251 (1985).

Les IC 50 (concentrations qui inhibent 50% de la liaison de Ro5-4864 tritié dans les membranes de rein) des composés de l'invention vont de 0,01 à 100 nanomolaires.

Les composés peuvent donc être utiles dans les domaines suivants :

— dans le système immunitaire comme immunomodulateurs tels que immunostimulateurs ou immunodépresseurs

— dans la régulation de la prolifération cellulaire

— dans le système cardiovasculaire comme vasodilatateurs coronariens et/ou protecteurs dans l'ischémie cardiaque

— dans le système nerveux central (action sur les mécanismes de défense tissulaire et le contrôle de la régénérescence des zones affectées après lésions de diverses origines)

— dans le système bronchopulmonaire comme bronchodilatateurs

— en dermatologie (régulation de la prolifération cellulaire dans la couche proliférative de l'épiderme et modulation de l'activité des glandes sébacées).

Les composés de l'invention peuvent être présentés sous toute forme appropriée pour l'administration par voie orale ou parentérale ou topique, par exemple sous forme de comprimés, de dragées, de pommades, de gélules, de solutions buvables ou injectables etc.. avec tout excipient approprié.

La posologie quotidienne peut aller de 0,5 à 2000 mg.

Annexe 1

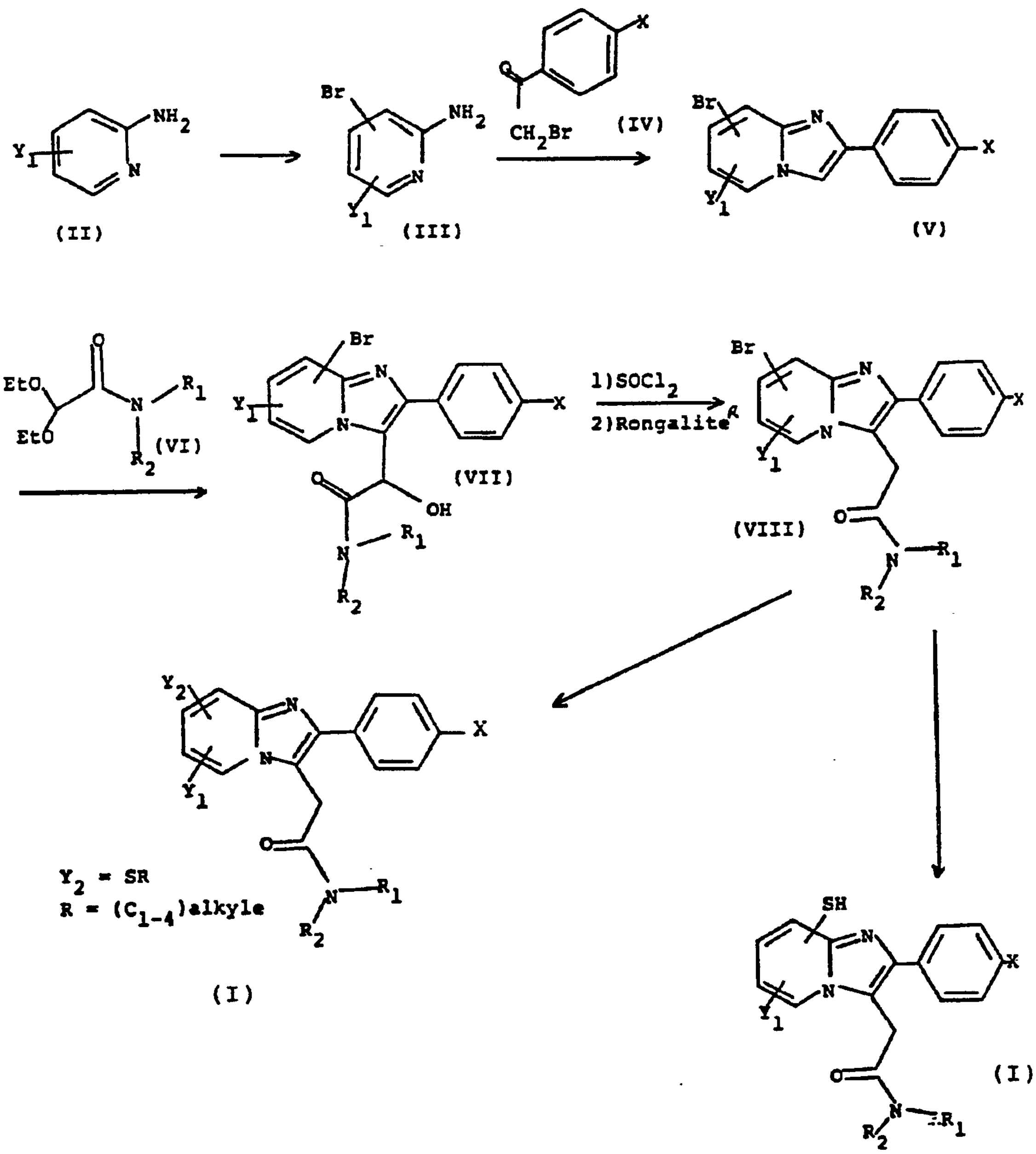

Annexe 2

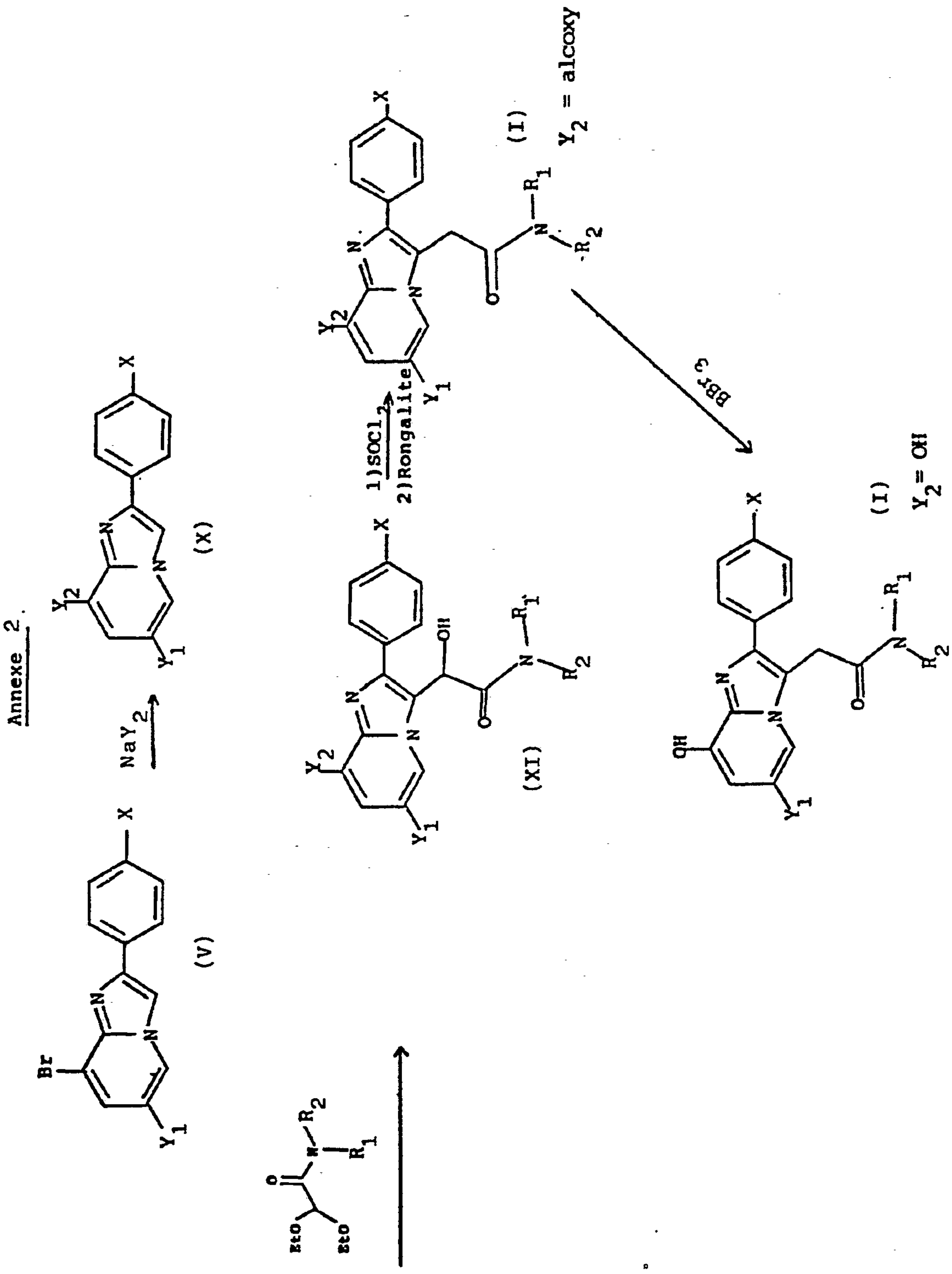


(V)

(X)

(XI)

(I) $Y_2$ = alcoxy

(I) $Y_2$ = OH

Annexe 3

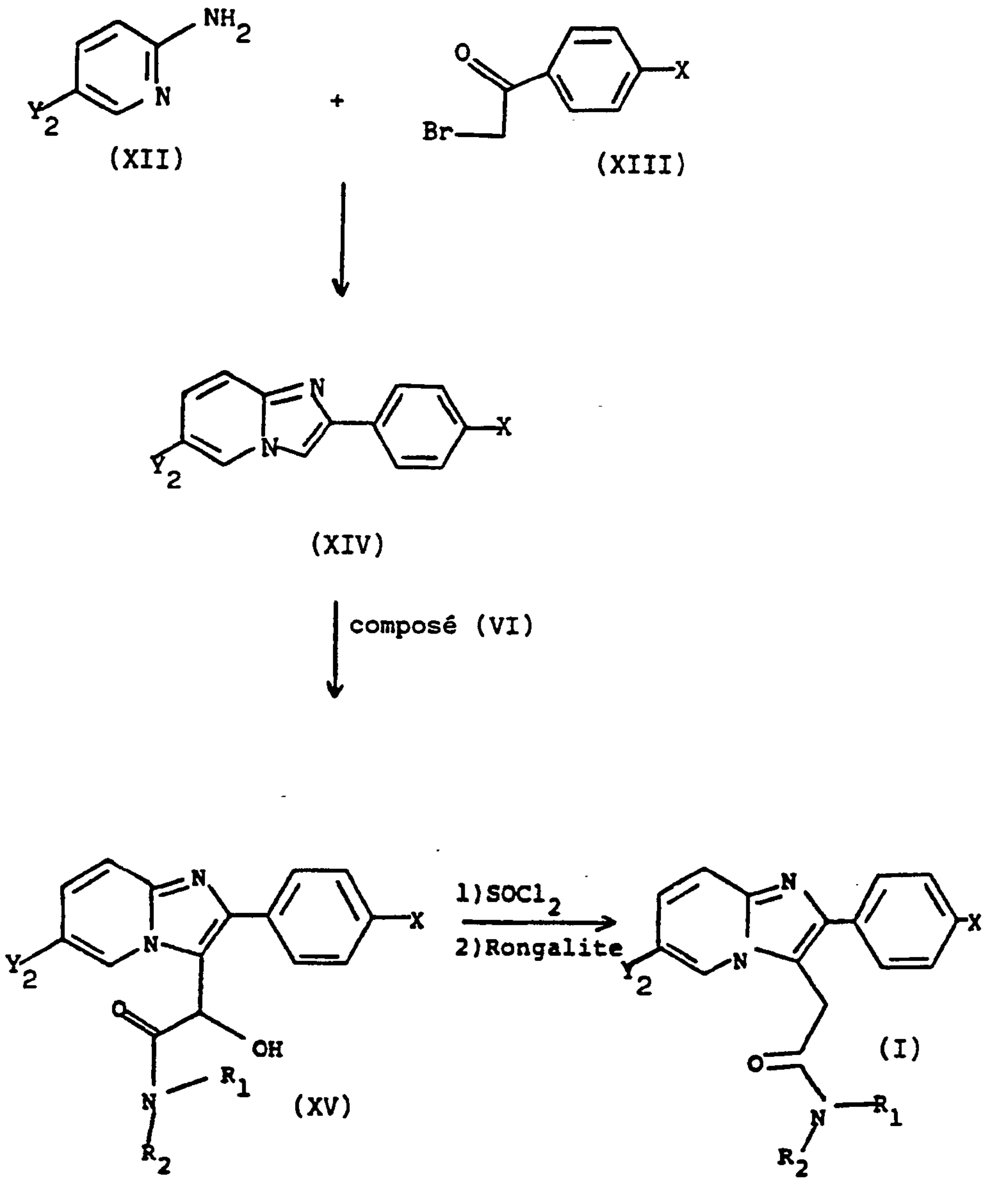


**Revendications**

**Revendications pour les Etats contractants suivants : AT, BE, CH, DE, FR, GB, IT, LI, LU, NL, SE.**

1. Dérivés d'imidazo[1,2-a]pyridines répondant à la formule (I)

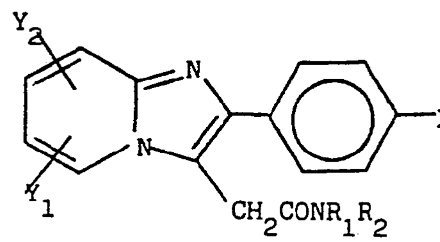

(I)

dans laquelle

- soit $Y_1$ représente un atome d'halogène,
  $Y_2$ représente soit un radical SR dans lequel R est H ou un radical $(C_{1-4})$alkyle ou un radical (alcoxy-4 phényl)méthyle, soit un radical hydroxy, soit un radical $(C_{1-4})$alcoxy ;
  X est un atome d'halogène,
  et $R_1$ et $R_2$ représentent chacun, indépendamment l'un de l'autre, soit un atome d'hydrogène, soit un radical $(C_{1-4})$alkyle droit ou ramifié,
- soit $Y_1$ représente un atome d'hydrogène,
  $Y_2$ représente un radical SR dans lequel R est H ou un radical $(C_{1-4})$alkyle ou un radical (alcoxy-4 phényl) méthyle, soit un radical hydroxy, soit un radical $(C_{1-4})$alcoxy ;
  X est un atome d'halogène ou un radical $(C_{1-4})$alkyle,
  et $R_1$ et $R_2$ représentent chacun, indépendamment l'un de l'autre, soit un atome d'hydrogène, soit un radical $(C_{1-4})$alkyle droit ou ramifié.

2. Dérivés selon la revendication 1, caractérisés par le fait que
$Y_1$ est en position 6 et représente un atome d'halogène,
$Y_2$ est en position 8 et représente soit un radical SR dans lequel R est H, un radical $(C_{1-4})$alkyle ou un radical (alcoxy-4 phényl)méthyle, soit un radical hydroxy, soit un radical $(C_{1-4})$alcoxy ;
X est un atome d'halogène,
$R_1$ et $R_2$ représentent chacun, indépendamment l'un de l'autre, soit un atome d'hydrogène, soit un radical $(C_{1-4})$alkyle droit ou ramifié.

3. Dérivés selon la revendication 2, caractérisés par le fait que
$Y_1$ est en position 6 et représente un atome de chlore,
$Y_2$ est en position 8 et représente un radical méthylthio, mercapto, méthoxy, hydroxy, n-butylthio ou (méthoxy-4 phényl)méthylthio,
X est un atome de chlore,
$R_1$ et $R_2$ représentent chacun le radical méthyle ou le radical n-propyle.

4. Dérivés selon la revendication 1, caractérisés par le fait que
$Y_1$ est H,
$Y_2$ est en position 6 et représente un radical Salkyle ou alcoxy,
X est un atome d'halogène ou un radical $(C_{1-4})$alkyle,
$R_1$ et $R_2$ représentent chacun, indépendamment l'un de l'autre, soit un atome d'hydrogène, soit un radical $(C_{1-4})$ alkyle droit ou ramifié.

5. Dérivés selon la revendication 4, caractérisés par le fait que
$Y_1$ est H,
$Y_2$ est en position 6 et représente le radical méthylthio ou méthoxy,
X est un atome de chlore ou le radical méthyle,
$R_1$ et $R_2$ représentent chacun le radical méthyle ou le radical n-propyle.

6. Médicament caractérisé en ce qu'il contient un composé spécifié dans l'une quelconque des revendications 1 à 5.

7. Composition pharmaceutique caractérisée en ce qu'elle contient un composé spécifié dans l'une quelconque des revendications 1 à 5 en association avec tout excipient approprié.

**Revendications pour les Etats contractants suivants : ES, GR**

1. Procédé de préparation de dérivés d'imidazo[1,2-a]pyridines répondant à la formule (I)

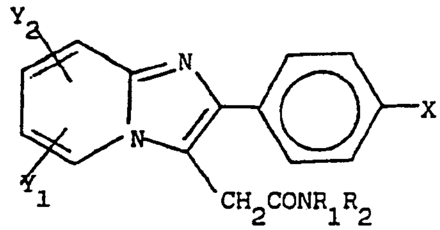

(I)

dans laquelle

- soit $Y_1$ représente un atome d'halogène,
  $Y_2$ représente soit un radical SR dans lequel R est H ou un radical $(C_{1-4})$alkyle ou un radical (alcoxy-4 phényl) méthyle, soit un radical hydroxy, soit un radical $(C_{1-4})$alcoxy ;
  X est un atome d'halogène,
  et $R_1$ et $R_2$ représentent chacun, indépendamment l'un de l'autre, soit un atome d'hydrogène, soit un radical $(C_{1-4})$alkyle droit ou ramifié,
- soit $Y_1$ représente un atome d'hydrogène,
  $Y_2$ représente un radical SR dans lequel R est H ou un radical $(C_{1-4})$alkyle ou un radical (alcoxy-4 phényl-)méthyle, soit un radical hydroxy, soit un radical $(C_{1-4})$alcoxy ;
  X est un atome d'halogène ou un radical $(C_{1-4})$alkyle,
  et $R_1$ et $R_2$ représentent chacun, indépendamment l'un de l'autre, soit un atome d'hydrogène, soit un radical $(C_{1-4})$alkyle droit ou ramifié,

procédé caractérisé en ce qu'on brome une amino-2 pyridine de formule (II)

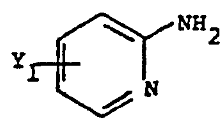

(II)

puis on fait réagir le composé bromé (III)

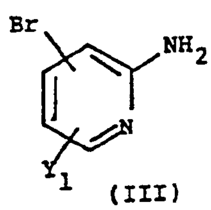

(III)

avec une bromocétone de formule (IV)

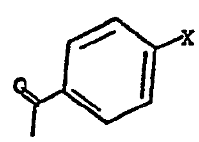

(IV)

on fait ensuite réagir l'imidazopyridine obtenue de formule (V)

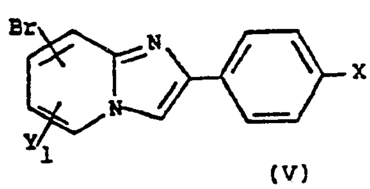

(V)

avec un acétal de formule (VI)

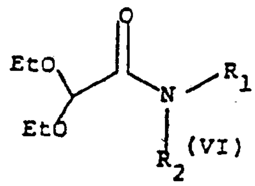

(VI)

on traite le composé obtenu de formule (VII)

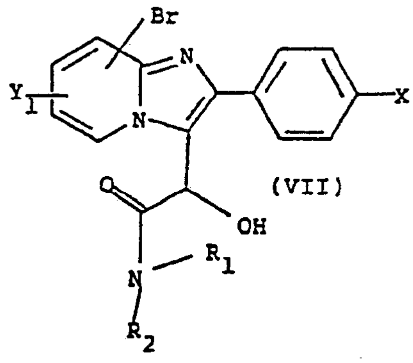

(VII)

par $SOCl_2$ puis on réduit le composé chloré obtenu in situ avec un agent tel que la "Rongalite®" ; on fait réagir le composé (VIII)

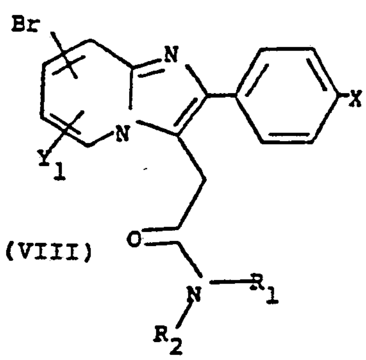

(VIII)

avec un alkylthiolate de sodium ; puis, pour obtenir les dérivés (I) dans lesquels $Y_2$ est SH, on fait réagir le composé (VIII) avec le méthoxy-4 benzèneméthanethiol, dans du diméthylformamide, en présence d'hydrure de sodium et on agite le composé obtenu à 0°C, dans de l'acide trifluoroacétique, en présence d'acétate de mercure, pour obtenir le thiol (I) sous forme de thiolate de mercure ou de disulfure, obtenu à partir du thiolate de mercure, par action de l'acide trifluoroacétique et de sulfure d'hydrogène.

2. Procédé de préparation d'imidazo[1,2-a]pyridines répondant à la formule (I)

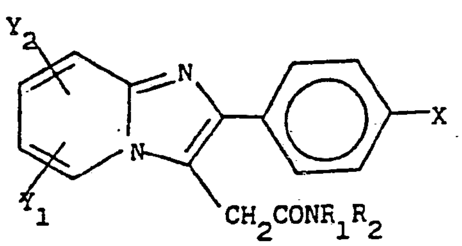

(I)

dans laquelle

$Y_1$ est en position 6 et représente un atome d'halogène,

$Y_2$ est en position 8 et représente soit un radical hydroxy, soit un radical $(C_{1-4})$alcoxy ;
X est un atome d'halogène,
$R_1$ et $R_2$ représentent chacun, indépendamment l'un de l'autre, soit un atome d'hydrogène, soit un radical $(C_{1-4})$ alkyle droit ou ramifié,
procédé caractérisé en ce qu'on fait réagir un composé (V)

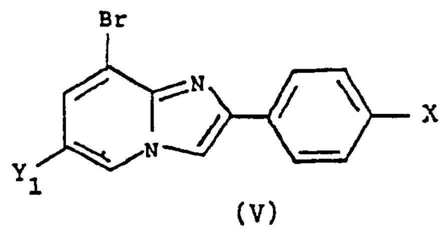
(V)

avec du méthylate de sodium dans de l'hexaméthylphosphorotriamide, puis on fait réagir le composé (X)

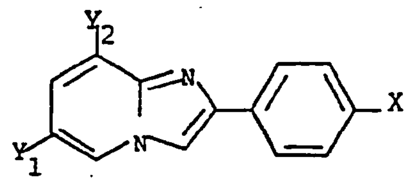
(X)

avec du diéthoxy-1,1 N,N-$R_1R_2$ acétamide, puis on traite le composé obtenu (XI)

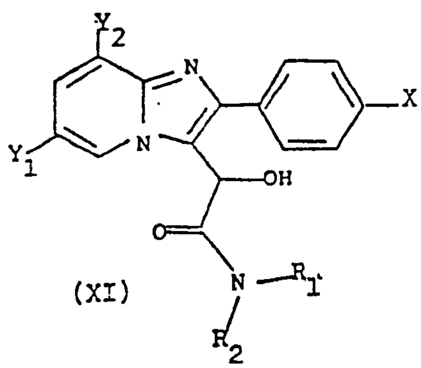
(XI)

par $SOCl_2$ puis par de la "Rongalite®" pour obtenir le composé alcoxylé (I) puis,si on le désire, on transforme le composé (I) alcoxylé en composé (I) hydroxylé ($Y_2$ = OH) par action de $BBr_3$.

3. Procédé de préparation d'imidazo[1,2-a]pyridines répondant à la formule (I)

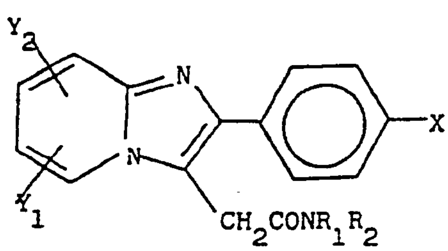
(I)

dans laquelle
$Y_1$ est H,
$Y_2$ est en position 6 et représente un radical Salkyle ou alcoxy,
X est un atome d'halogène ou un radical $(C_{1-4})$alkyle,
$R_1$ et $R_2$ représentent chacun, indépendamment l'un de l'autre, soit un atome d'hydrogène, soit un radical

($C_{1-4}$) alkyle droit ou ramifié,
procédé caractérisé en ce qu'on condense une amino-2 pyridine de formule (XII)

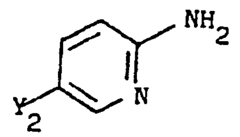

(XII)

avec une α-bromo acétophénone de formule (XIII)

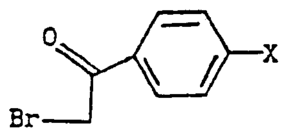

(XIII)

puis on condense l'imidazopyridine obtenue de formule (XIV)

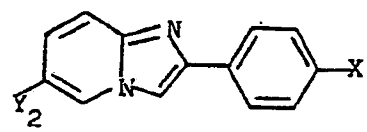

(XIV)

avec l'acétal de glyoxamide de formule (VI)

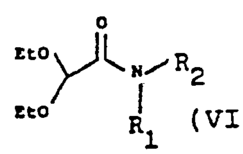

(VI)

on traite l'α-hydroxyamide (XV)

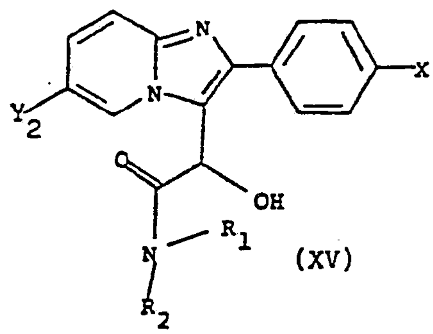

(XV)

par $SOCl_2$ puis on réduit le composé obtenu à l'aide de "Rongalite®" pour obtenir le composé de formule (I).

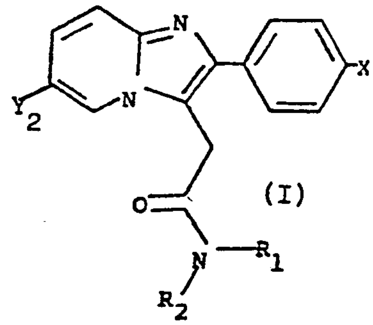

(I)

**Patentansprüche**

**Patentansprüche für folgende Vertragsstaaten : AT, BE, CH, DE, FR, GB, IT, LI, LU, NL, SE**

1. Imidazo[1,2-a]pyridin-Derivate der Formel (I)

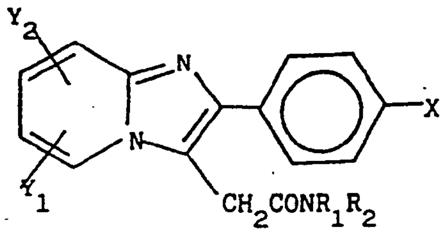

(I)

in welcher

- $Y_1$ ein Halogenatom, $Y_2$ einen Rest SR, in welchem R für H oder einen $(C_{1-4})$-Alkyl- oder einen (4-Alkoxyphenyl)-methylrest steht, einen Hydroxy- oder einen $(C_{1-4})$-Alkoxyrest, X ein Halogenatom und $R_1$ und $R_2$ jeweils unabhängig voneinander ein Wasserstoffatom oder einen geradkettigen oder verzweigten $(C_{1-4})$Alkylrest bedeuten, oder
- $Y_1$ ein Wasserstoffatom, $Y_2$ einen Rest SR, in welchem R für H oder einen $(C_{1-4})$-Alkyl- oder einen (4-Alkoxyphenyl)-methylrest steht, einen Hydroxy- oder einen $(C_{1-4})$-Alkoxyrest, X ein Halogenatom oder einen $(C_{1-4})$-Alkylrest und $R_1$ und $R_2$ jeweils unabhängig voneinander ein Wasserstoffatom oder einen geradkettigen oder verzweigten $(C_{1-4})$-Alkylrest bedeuten.

2. Derivate nach Anspruch 1, dadurch gekennzeichnet, daß $Y_1$ in Position 6 steht und ein Halogenatom darstellt, $Y_2$ in Position 8 steht und einen Rest SR, in welchem R für H, einen $(C_{1-4})$-Alkyl- oder einen (4-Alkoxyphenyl)-methylrest steht, einen Hydroxy- oder einen $(C_{1-4})$-Alkoxyrest, X ein Halogenatom und $R_1$ und $R_2$ jeweils unabhängig voneinander ein Wasserstoffatom oder einen geradkettigen oder verzweigten $(C_{1-4})$-Alkylrest bedeuten.

3. Derivate nach Anspruch 2, dadurch gekennzeichnet, daß $Y_1$ in Position 6 steht und ein Chloratom darstellt, $Y_2$ in Position 8 steht und einen Methylthio-, Mercapto-, Methoxy-, Hydroxy-, n-Butylthio- oder (4-Methoxyphenyl)-methylthiorest, X ein Chloratom und $R_1$ und $R_2$ jeweils den Methylrest oder den n-Propylrest bedeuten.

4. Derivate nach Anspruch 1, dadurch gekennzeichnet, daß $Y_1$ für H steht, $Y_2$ in Position 6 steht und einen Salkyl- oder Alkoxyrest darstellt, X ein Halogenatom oder einen $(C_{1-4})$-Alkylrest bedeutet und $R_1$ und $R_2$ unabhängig voneinander entweder ein Wasserstoffatom oder einen geradkettigen oder verzweigten $(C_{1-4})$-Alkylrest darstellen.

5. Derivate nach Anspruch 4, dadurch gekennzeichnet, daß $Y_1$ für Wasserstoff steht, $Y_2$ in Position 6 steht und den Methylthio- oder Methoxyrest darstellt, X ein Chloratom oder den Methylrest bedeutet und $R_1$ und $R_2$ den Methylrest oder den n-Propylrest darstellen.

6. Arzneimittel, dadurch gekennzeichnet, daß es eine in einem der Ansprüche 1 bis 5 dargelegte Verbindung enthält.

7. Pharmazeutische Zusammensetzung, dadurch gekennzeichnet, daß sie eine in einem der Ansprüche

1 bis 5 dargelegte Verbindung in Kombination mit irgendeinem geeigneten Träger enthält.

**Patentansprüche für folgende Vertragsstaaten : ES, GR**

1. Verfahren zur Herstellung von Imidazo[1,2-a]pyridin-Derivaten der Formel (I)

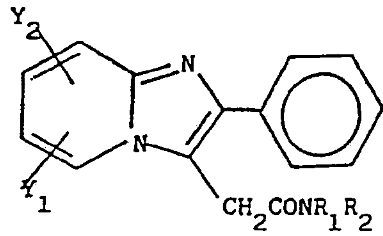

(I)

in welcher

- $Y_1$ ein Halogenatom, $Y_2$ einen Rest SR, in welchem R für H oder einen $(C_{1-4})$-Alkyl- oder einen (4-Alkoxyphenyl)-methylrest steht, einen Hydroxy- oder einen $(C_{1-4})$-Alkoxyrest, X ein Halogenatom und $R_1$ und $R_2$ jeweils unabhängig voneinander ein Wasserstoffatom oder einen geradkettigen oder verzweigten $(C_{1-4})$-Alkylrest bedeuten, oder
- $Y_1$ ein Wasserstoffatom,
$Y_2$ einen Rest SR, in welchem R für H oder einen $(C_{1-4})$-Alkyl- oder einen (4-Alkoxyphenyl)-methylrest steht, einen Hydroxyrest oder einen $(C_{1-4})$-Alkoxyrest,
X ein Halogenatom oder einen $(C_{1-4})$-Alkylrest und
$R_1$ und $R_2$ jeweils unabhängig voneinander ein Wasserstoffatom oder einen geradkettigen oder verzweigten $(C_{1-4})$-Alkylrest bedeuten,

welches Verfahren dadurch gekennzeichnet ist, daß man ein 2-Aminopyridin der Formel (II)

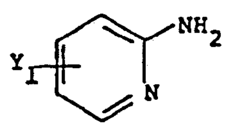

(II)

bromiert, dann die bromierte Verbindung (III)

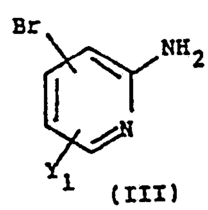

(III)

mit einem Bromketon der Formel (IV)

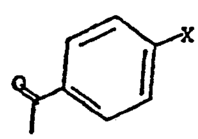

(IV)

zur Reaktion bringt, anschließend das erhaltene Imidazopyridin der Formel (V)

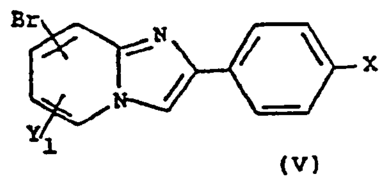

(V)

mit einem Acetal der Formel (VI)

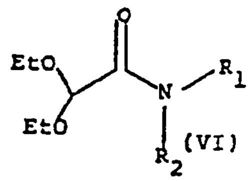

(VI)

umsetzt, die erhaltene Verbindung der Formel (VII)

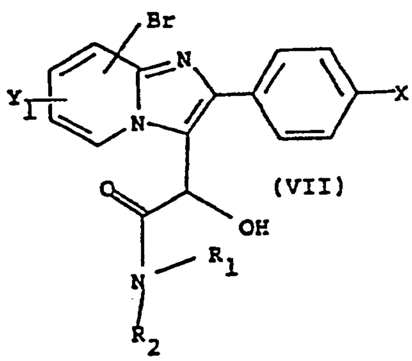

(VII)

mit $SOCl_2$ behandelt, dann die erhaltene chlorierte Verbindung in situ mit einem Mittel, wie "Rongalite$^R$" reduziert, anschließend die Verbindung (VIII)

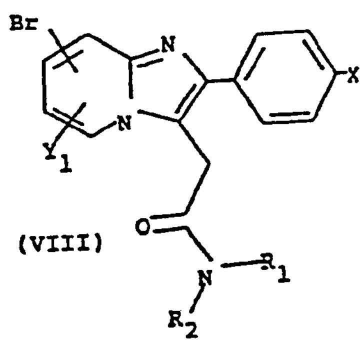

(VIII)

mit einem Natrium-Alkylthiolat reagieren läßt und schließlich, zur Herstellung der Derivate (I), in welchen $Y_2$ für SH steht, die Verbindung (VIII) mit 4-Methoxybenzolmethanthiol in Dimethylformamid in Gegenwart von Natriumhydrid umsetzt und die erhaltene Verbindung bei 0°C in Trifluoressigsäure in Gegenwart von Quecksilberacetat rührt, um das Thiol (I) in Form des Quecksilberthiolats zu erhalten, oder um das Disulfid zu gewinnen, das aus dem Quecksilberthiolat durch Einwirkung von Trifluoressigsäure und Schwefelwasserstoff gewonnen wird.

2. Verfahren zur Herstellung von Imidazo[1,2-a]pyridin-Derivaten der Formel (I)

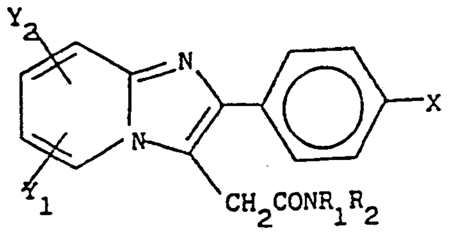

(I)

in welcher $Y_1$ in Position 6 steht und für ein Halogenatom steht, $Y_2$ in Position 8 steht und einen Hydroxy- oder einen $(C_{1-4})$-Alkoxyrest bedeutet, X ein Halogenatom darstellt und $R_1$ und $R_2$ jeweils unabhängig voneinander für ein Wasserstoffatom oder einen geradkettigen oder verzweigten $(C_{1-4})$-Alkylrest steht, dadurch gekennzeichnet, daß man eine Verbindung (V)

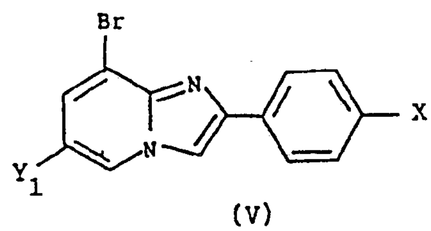

(V)

mit Natriummethylat in Hexamethylphosphortriamid umsetzt, dann die Verbindung (X)

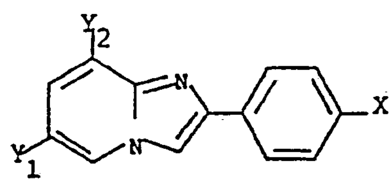

(X)

mit 1,1-Diethoxy-N,N-$R_1R_2$-acetamid reagieren läßt, dann die erhaltene Verbindung (XI)

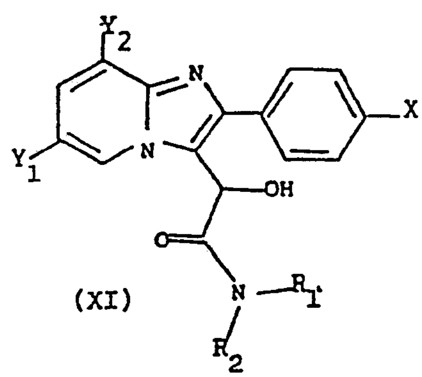

(XI)

mit $SOCl_2$ und anschließend mit "Rongalite[R]" behandelt, um die alkoxylierte Verbindung (I) zu erhalten, und weiters gewünschtenfalls die alkoxylierte Verbindung (I) durch Einwirkung von $BBr_3$ in die hydroxylierte Verbindung (I) ($Y_2$ = OH) umwandelt.

3. Verfahren zur Herstellung von Imidazo[1,2-a]pyridin-Derivaten der formel (I)

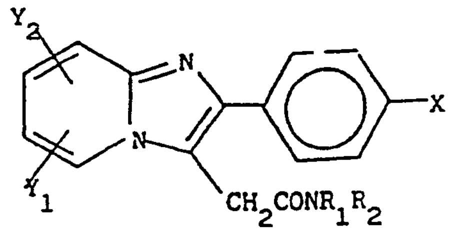

(I)

in welcher $Y_1$ für H steht, $Y_2$ in Position 6 steht und einen Salkyl- oder Alkoxyrest bedeutet, X ein Halogenatom oder einen $(C_{1-4})$-Alkylrest und $R_1$ und $R_2$ unabhängig voneinander entweder ein Wasserstoffatom oder einen geradkettigen oder verzweigten $(C_{1-4})$-Alkylrest darstellen, dadurch gekennzeichnet, daß man ein 2 -Aminopyridin der formel (XII)

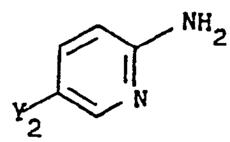

(XII)

mit einem alpha-Bromacetophenon der formel (XIII)

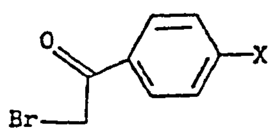

(XIII)

kondesiert, dann das erhaltene Imidazopyridin der Formel (XIV)

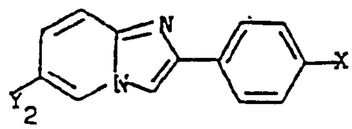

(XIV)

mit dem Glyoxamidacetal der Formel (VI)

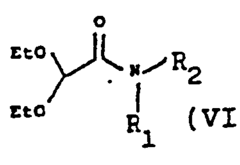

(VI)

kondensiert, dann das alpha-Hydroxyamid (XV)

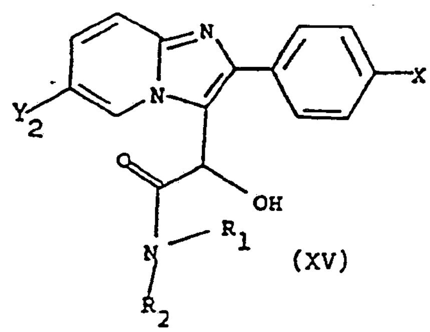

(XV)

mit $SOCl_2$ behandelt und anschließend die erhaltene Verbindung mit Hilfe von "Rongalite[R]" reduziert, um die Verbindung der Formel (I)

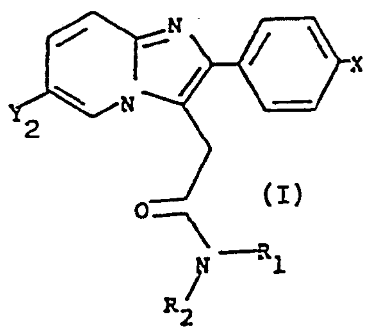

(I)

zu gewinnen.

## Claims

### Claims for the following Contracting States : AT, BE, CH, DE, FR, GB, IT, LI, LU, NL, SE

1. Imidazo[1,2-a]pyridine derivatives corresponding to the formula (I)

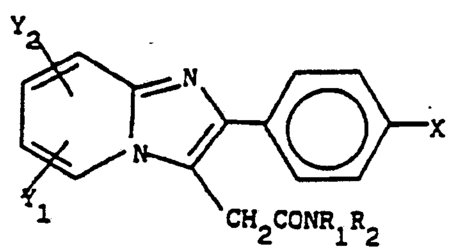

(I)

in which

- either $Y_1$ denotes a halogen atom,
  $Y_2$ either denotes a radical SR in which R is H or a $(C_{1-4})$ alkyl radical or a (4-alkoxyphenyl)methyl radical, or denotes a hydroxyl radical or a $(C_{1-4})$ alkoxy radical ;
  X is a halogen atom,
  and $R_1$ and $R_2$ each denote, independently of one another, either a hydrogen atom or a linear or branched $(C_{1-4})$ alkyl radical,
- or $Y_1$ denotes a hydrogen atom,
  $Y_2$ denotes a radical SR in which R is H or a $(C_{1-4})$ alkyl radical or a (4-alkoxyphenyl)methyl radical, or denotes a hydroxyl radical or a $(C_{1-4})$ alkoxy radical ;
  X is a halogen atom or a $(C_{1-4})$ alkyl radical, and
  $R_1$ and $R_2$ each denote, independently of one another, either a hydrogen atom or a linear or branched $(C_{1-4})$ alkyl radical.

2. Derivatives according to Claim 1, characterised in that
$Y_1$ is at the 6-position and denotes a halogen atom,
$Y_2$ is at the 8-position and either denotes a radical SR in which R is H, a $(C_{1-4})$ alkyl radical or a (4-alkoxyphenyl) methyl radical, or denotes a hydroxyl radical or a $(C_{1-4})$ alkoxy radical ;
X is a halogen atom, and
$R_1$ and $R_2$ each denote, independently of one another, either a hydrogen atom or a linear or branched $(C_{1-4})$ alkyl radical.

3. Derivatives according to Claim 2, characterised in that
$Y_1$ is at the 6-position and denotes a chlorine atom,
$Y_2$ is at the 8-position and denotes a methylthio, mercapto, methoxy, hydroxyl, n-butylthio or (4-methoxyphenyl)methylthio radical,
X is a chorine atom, and
$R_1$ and $R_2$ each denote a methyl radical or an n-propyl radical.

4. Derivatives according to Claim 1, characterised in that
$Y_1$ is H,
$Y_2$ is at the 6-position and denotes an S-alkyl or alkoxy radical,
X is a halogen atom or a $(C_{1-4})$ alkyl radical, and
$R_1$ and $R_2$ each denote, independently of one another, either a hydrogen atom or a linear or branched $(C_{1-4})$ alkyl radical.

5. Derivatives according to Claim 4, characterised in that
$Y_1$ is H,
$Y_2$ is at the 6-position and denotes a methylthio or methoxy radical,
X is a chlorine atom or a methyl radical, and
$R_1$ and $R_2$ each denote a methyl radical or an n-propyl radical.

6. Medicinal product, characterised in that it contains a compound specified in any one of Claims 1 to 5.

7. Pharmaceutical composition, characterised in that it contains a compound specified in any one of Claims 1 to 5, in combination with any suitable excipient.

**Claims for the following Contracting States : ES, GR**

1. Process for preparing imidazo[1,2-a]pyridine derivatives corresponding to the formula (I)

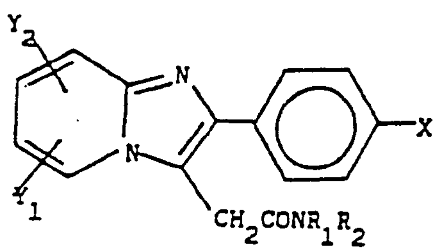

(I)

in which

• either $Y_1$ denotes a halogen atom,
$Y_2$ either denotes a radical SR in which R is H or a $(C_{1-4})$ alkyl radical or a (4-alkoxyphenyl)methyl radical, or denotes a hydroxyl radical or a $(C_{1-4})$ alkoxy radical ;
X is a halogen atom,
and $R_1$ and $R_2$ each denote, independently of one another, either a hydrogen atom or a linear or branched $(C_{1-4})$ alkyl radical,

• or $Y_1$ denotes a hydrogen atom,
$Y_2$ denotes a radical SR in which R is H or a $(C_{1-4})$ alkyl radical or a (4-alkoxyphenyl)methyl radical, or denotes a hydroxyl radical or a $(C_{1-4})$ alkoxy radical ;
X is a halogen atom or a $(C_{1-4})$ alkyl radical, and
$R_1$ and $R_2$ each denote, independently of one another, either a hydrogen atom or a linear or branched $(C_{1-4})$ alkyl radical, which process is characterised in that a 2-aminopyridine of formula (II)

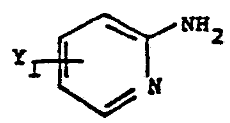

(II)

is brominated, the bromo compound (III)

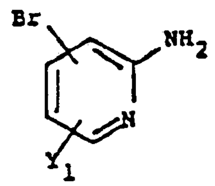

(III)

is then reacted with a bromo ketone of formula (IV)

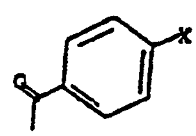

(IV)

the imidazopyridine of formula (V) obtained

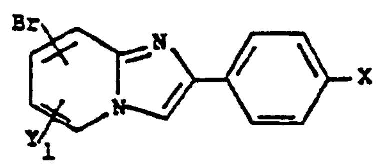

(V)

is thereafter reacted with an acetal of formula (VI)

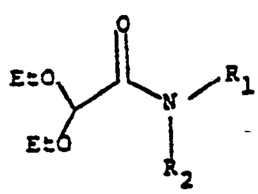

(VI)

the compound of formula (VII) obtained

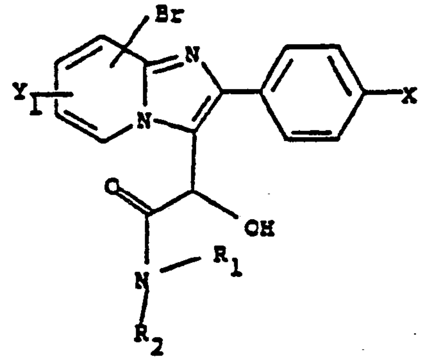

(VII)

is treated with $SOCl_2$, and the chloro compound obtained is then reduced in situ with an agent such as "Ron-

galite®" ; the compound (VIII)

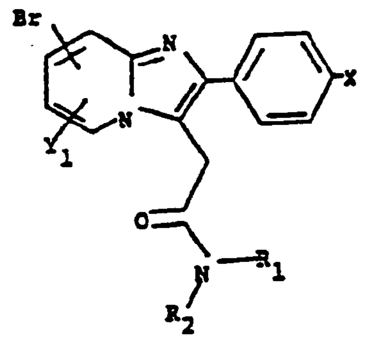

(VIII)

is reacted with a sodium alkylthiolate ;
then, to obtain the derivatives (I) in which $Y_2$ is SH, the compound (VIII) is reacted with 4-methoxybenzenemethanethiol in dimethylformamide in the presence of sodium hydride, and the compound obtained is stirred at 0°C in trifluoroacetic acid in the presence of mercury acetate to obtain the thiol (I) in the form of a mercury thiolate or a disulphide, obtained from the mercury thiolate by the action of trifluoroacetic acid and hydrogen sulphide.

2. Process for preparing imidazo[1,2-a]pyridines corresponding to the formula (I)

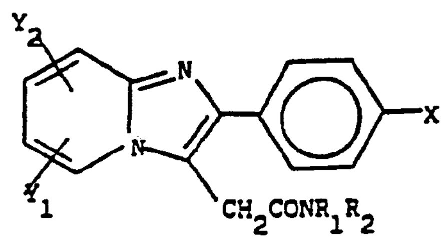

(I)

in which

$Y_1$ is at the 6-position and denotes a halogen atom,
$Y_2$ is at the 8-position and denotes either a hydroxyl radical or a $(C_{1-4})$ alkoxy radical ;
X is a halogen atom, and
$R_1$ and $R_2$ each denote, independently of one another, either a hydrogen atom or a linear or branched $(C_{1-4})$ alkyl radical,
which process is characterised in that a compound (V)

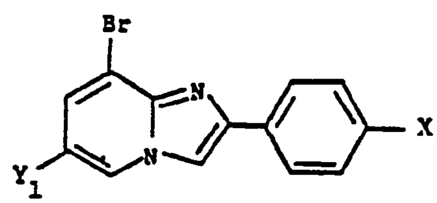

(V)

is reacted with sodium methylate in hexamethylphosphorotriamide, the compound (X)

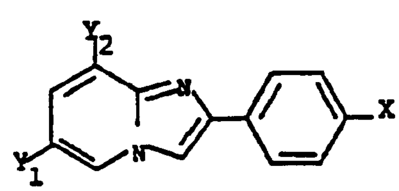

(X)

is then reacted with 1,1-diethoxy-N,N-($R_1R_2$)acetamide, the compound obtained (XI)

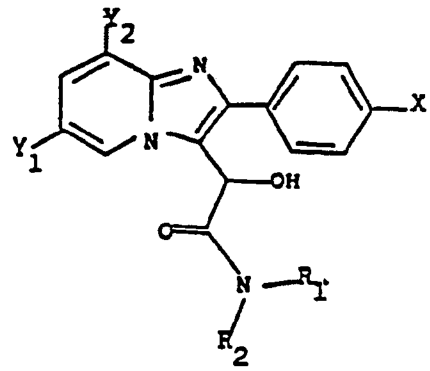

(XI)

is then treated with $SOCl_2$ and then with "Rongalite®" to obtain the alkoxylated compound (I) and then, if so desired, the alkoxylated compound (I) is converted to a hydroxylated compound (I) ($Y_2$ = OH) by the action of $BBr_3$.

3. Process for preparing imidazo[1,2-a]pyridines corresponding to the formula (I)

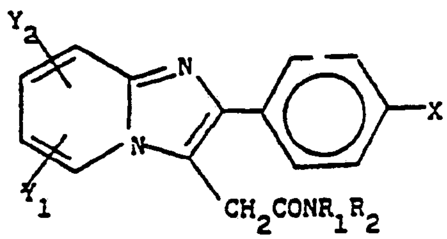

(I)

in which

$Y_1$ is H,
$Y_2$ is at the 6-position and denotes an S-alkyl or alkoxy radical,
X is a halogen atom or a $(C_{1-4})$ alkyl radical, and
$R_1$ and $R_2$ each denote, independently of one another, either a hydrogen atom or a linear or branched $(C_{1-4})$ alkyl radical,
which process is characterised in that a 2 -aminopyridine of formula (XII)

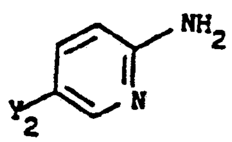

(XII)

is condensed with an α-bromoacetophenone of formula (XIII)

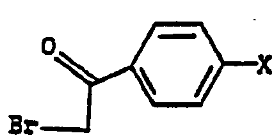

(XIII)

and the imidazopyridine of formula (XIV) obtained

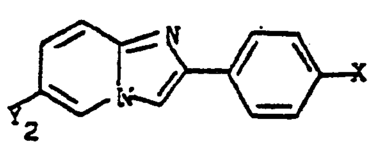

(XIV)

is then condensed with the glyoxamide acetal of formula (VI)

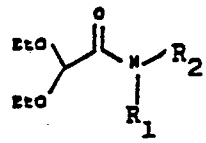

(VI)

the α-hydroxyamide (XV)

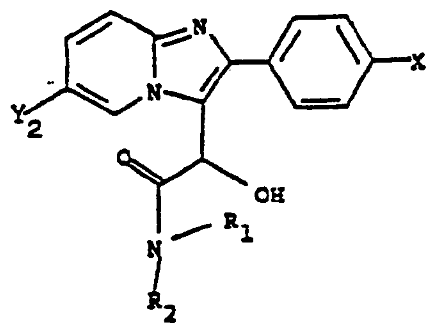

(XV)

is treated with $SOCl_2$, and the compound obtained is then reduced using "Rongalite®" to obtain the compound of formula (I)

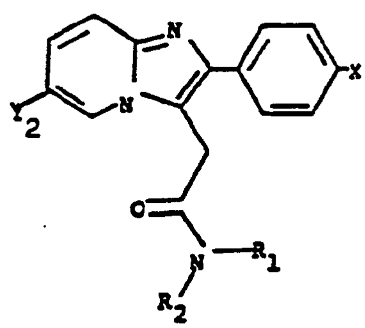

(I)